Europäisches Patentamt

**European Patent Office**

Office européen des brevets

(11) Publication number: **0 187 728**
**B1**

(12) # EUROPEAN PATENT SPECIFICATION

(45) Date of publication of the patent specification: 04.10.89

(51) Int. Cl.⁴: **A 41 B 13/02**

(21) Application number: 86400040.1

(22) Date of filing: 09.01.86

(54) Disposable underpants, such as child's training pants and the like.

(30) Priority: 10.01.85 US 690351
31.05.85 US 740135
16.12.85 US 807900

(43) Date of publication of application:
16.07.86 Bulletin 86/29

(45) Publication of the grant of the patent:
04.10.89 Bulletin 89/40

(84) Designated Contracting States:
AT BE CH DE FR IT LI LU NL SE

(56) References cited
FR-A-2 124 196
FR-A-2 229 383
US-A-3 424 162
US-A-4 205 679
US-A-4 333 463

(73) Proprietor: KIMBERLY- CLARK CORPORATION, 401 North Lake Street, Neenah Wisconsin 54956 (US)

(72) Inventor: Heran, William M., 106 Limekiln Drive, Neenah Wisconsin (US)
Inventor: Fleischer, Glen R., 533 Kessler Drive, Neenah Wisconsin (US)
Inventor: Damico, Joyce Ann, 1219 Green Acres Lane, Neenah Wisconsin (US)
Inventor: Van Gompel, Paul Theodore, route 2, Box 227, Hortonville Wisconsin (US)
Inventor: Van Deurzen, John Irvin, 224 Fredrick Street, Menasha Wisconsin (US)
Inventor: Strohbeen, David Thomas, 106 Cherry Court, Appleton Wisconsin (US)

(74) Representative: Sauvage, Renée, Cabinet Sauvage 100 bis, avenue de Saint- Mandé, F-75012 Paris (FR)

EP 0 187 728 B1

## Description

### Technical Field

This invention relates generally to the field of disposable underpants having elasticized leg and waist openings, particularly disposable child's training pants and similar garments.

### Background Art

Disposable diapers, as is well known, now find wide-spread use for infant care and have generally replaced the use of cloth diapers. The typical disposable diaper is a three-layer composite structure comprising a liquid permeable bodyside inner liner, a liquid impermeable outer cover and an absorbent batt sandwiched between the liner and the cover. Materials now in general use for the three principal elements of a disposable diaper include various types of nonwoven fabrics for the bodyside liner, a thin thermoplastic film for the outer cover and cellulosic fluff for the absorbent batt.

Disposable diapers of the type presently on the market are flat open-sided garments that are intended to be fit about an infant by a parent while the infant is lying down.

The rear panel of the diaper is placed underneath the infant, and the front panel drawn between the infant's legs, after which the sides are overlapped and held together by pressure sensitive adhesive tape. A diaper is meant for use when the child is young and dependent upon a parent for this essential purpose.

The popularity of disposable diapers indicates a demand for a disposable underpant, such as a disposable training pant that can be used when a child grows out of a diaper. Diapers are typically used with infants up to about fifteen months old. When a child reaches an age in the range of about fifteen to thirty months, however, a parent generally desires to start toilet training so that the child can become independent of a parent. The training pant is intended for use when the child has reached an age at which he or she is ready to graduate to an underpant type of garment as a replacement for disposable diapers previously used. Thus, a suitable training pant must be a garment having closed sides so that a child can raise and lower it as necessary without requiring the aid of a parent. At the same time, a training pant must provide features of liquid and solid absorbency and prevent leakage of the waste fluids.

Cloth training pants are known, for example, from US-A-4 205 679. Although widely used, they have disadvantages. Current cloth training pants have very little absorbency and often must be used with exterior rubber or plastic pants. When a child wets a cloth training pant, most often all of the child's clothes must be changed. Further, if a child has a bowel movement, it is difficult to remove a cloth pant without making a mess, and the pant must be soaked and bleached. All of these factors can make the toilet training process frustrating for both child and parent.

In addition, it is believed that the psychology of the toilet training stage is such that the child should perceive he or she is graduating to a garment that is different than a disposable diaper. The requirements for a disposable underpant such as a training pant are not satisfactorily met by the constructions of disposable diapers as currently known in the art. In this connection, for example, the typical disposable diaper, as stated previously, has an outer layer comprising a liquid impermeable sheet of plastic film. Various techniques have been used to give the plastic sheet the feel and appearance of texture, but the exterior of the garment has a plastic feel or appearance which is closely associated with the concept of a diaper but would be inappropriate for a disposable training pant. Since the purpose of a training pant is to encourage the child to make the transition from diapers to washable or reusable cloth underpants, it is important that a disposable training pant simulate a cloth underpant as much as possible.

The need for a disposable training pant or similar underpant capable of meeting the demand for this type of garment has been the impetus for the development of the present invention.

### Summary of the Invention

An object of this invention is to overcome disadvantages of prior disposable underpants.

A further object of this invention is to create a discrete side seal for disposable garments.

A further object of this invention is to simplify the manufacture of disposable undergarments.

A further additional object of the invention is to provide strong garment side seals.

Our present invention provides a disposable underpant, particularly a disposable training pant, comprising a three-dimensional garment having closed side seams, an elasticized waist opening and elasticized leg openings, and including a bodyside liner and outer cover with an absorbent batt sandwiched therebetween, wherein the outer cover is a two layer composite material providing the dual characteristics of liquid imperviousness and a cloth-like appearance.

The formation process for the pant involves the necessity of formation of the garment with the interior facing outward for side sealing after which the garment is reversed prior to packaging for sale. Reversing of the completed elasticized garment is an extra mechanical handling step that it would be desirable to eliminate as it is complicated and expensive. However, generally undergarments have been formed with inner side seams seals as the looks is improved by not having flaps on the outer surface. Further, the turning of the flaps to the inside of the garment has created additional difficulties in that the inside seals can be a source of skin irritation unless they are

2

carefully formed. On the other hand, if exterior conventional seals are formed, they create flaps that have an undesirable appearance as it is intended that the garments resemble cloth underpants which do not have exterior seams. Therefore, there is a need for an exterior seal on a disposable garment that is discrete, strong, and easy to form.

In one embodiment, the side seam of the invention is particularly advantageous for disposable garments where low cost formation is especially desirable. The side seal of the invention is strong and further the seal is particularly effective for multi-layered garments, even providing seam adhesion when elastic is within the seam. Further, the multi-line bond provides a higher bond strength than one large seal at the edges. The multi-line sealing provides an attractive almost invisible edge. The formation of the outer seal does not provide a seam on the inside of the garment to irritate the wearer. These and other advantages will be apparent from the detailed description below.

This structure provides a disposable undergarment which is perceived as protective underwear for a child rather than a diaper associated for use by an infant. An undergarment to be suitable for use in toilet training children or by incontinent children or adults should comprise:

a) a non-woven fabric cover having a front portion, a rear portion and a crotch portion connecting the front and rear portions, both side margins of the front portion being joined to respective side margins of the rear portion so as to define a three-dimensional undergarment having a pair of leg openings and a waist opening;

b) a liquid pervious inner liner and an absorbent batt between the liner and the fabric cover, a first elastic means extending about one leg opening, a second elastic means extending about the other leg opening, and a third elastic means extending about the waist opening.

## Description of the Drawings

Fig. 1 is a perspective view of a disposable underpant according to the present invention;

Fig. 2 is a side perspective view of the disposable underpant of Fig. 1;

Fig. 3 is a horizontal sectional view of the disposable underpant of Fig. 1;

Fig. 4 is a partial horizontal sectional view similar to Fig. 3 illustrating an alternate form of side seam for the disposable underpant of Fig. 1;

Fig. 5 is a plan view, with portions broken away illustrating a flat blank as suitable for producing the underpant of Fig. 1;

Fig. 6 is a plan view, with portions broken away, illustrating another form of flat blank suitable for producing the underpant of Fig. 1;

Fig. 7 is a sectional view of a side seam in accordance with the invention.

Fig. 8 is a sectional view of a side seam at the waist of a disposable underpant in accordance with the invention.

Fig. 9 is a side perspective view of an alternate form of the disposable panty of Figure 1.

Fig. 10 is a partial sectional view illustrating one form of elasticized leg and/or waist opening of the underpant of Fig. 1;

Fig. 11 is a partial sectional view illustrating another form of elasticized leg and/or waist opening for the underpant of Fig. 1; and

Fig. 12 is a side perspective view of an alternate form of the disposable panty of Fig. 1.

## Detailed Description of the Invention

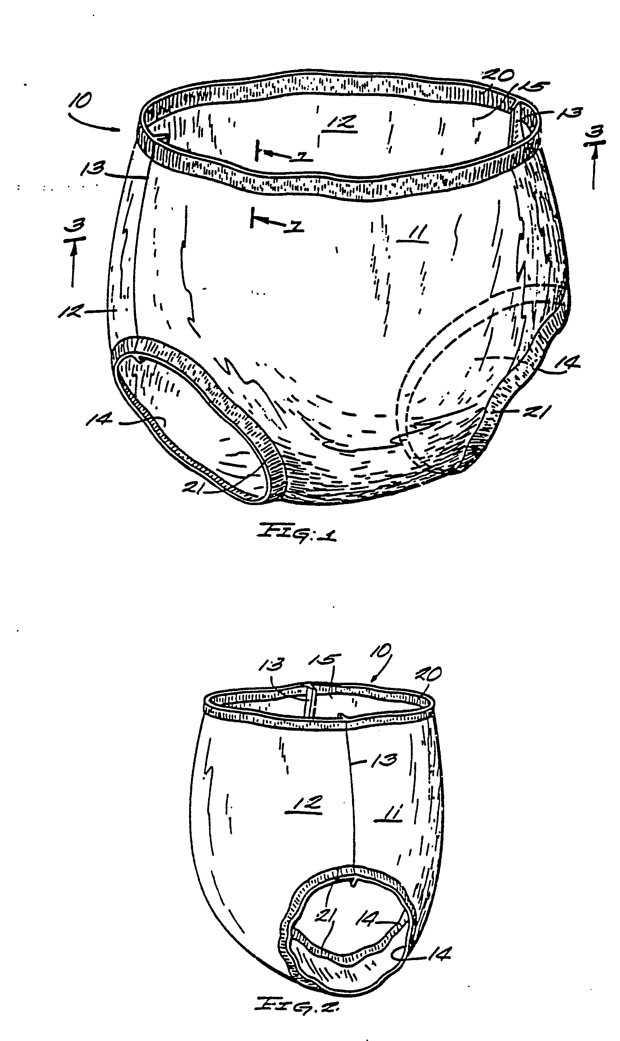

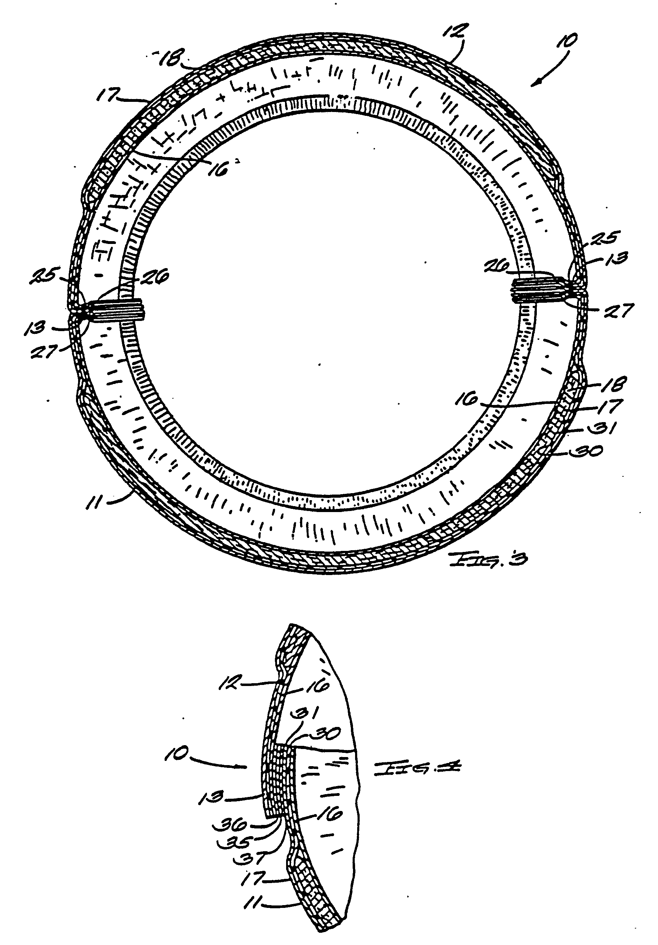

Figs. 1 and 2 illustrate, in front and side perspective views respectively, a disposable underpant 10 constructed in accordance with the present invention. The underpant 10 includes a front panel 11 and a rear panel 12 joined together alongside seams 13 to form a three-dimensional garment with closed sides having a pair of leg openings 14 and a waist opening 15. Referring now to Fig. 3, the underpant 10 includes a moisture pervious bodyside liner 16, a moisture impervious outer cover 17, and a absorbent batt 18 positioned between the liner 16 and outer cover 17. The absorbent batt may be secured to either the bodyside liner or the outer cover, or both, by any suitable means well known in the art such as lines or other patterns of adhesive, pressure sensitive tapes, heat seals, sonic seals, etc.

Referring again to Fig. 1, the waist opening 15 of the underpant 10 is surrounded by a circumferential elasticized band 20 and each leg opening 14 is surrounded by a circumferential elasticized band 21. Suitable constructions for the elasticized bands 20 and 21 are described in further detail later in this description.

The bodyside liner 16 can be any flexible porous sheet which passes fluids therethrough, i.e. a moisture pervious material, to be absorbed by the absorbent batt 18. The liner may comprise a nonwoven web or sheet of polyolefin fibers, such as polypropylene or polyethylene, or polyester fibers; a web of spun bonded polypropylene, polyethylene or polyester fibers; a web of rayon fibers; a bonded carded web of synthetic or natural fibers, or a mixture of synthetic and natural fibers, and the like. Further the liner 16 may also comprise a plastic film which is perforated or apertured to obtain the desired degree of moisture perviousness, and may also comprise an expanded plastic webbing material or a scrim material. The bodyside liner 16 preferably is made of a material which will feel soft and comfortable against the skin of an infant or adult.

The absorbent batt 18 may comprise any suitable material capable of absorbing and retaining waste fluids that pass through the liner 16. Thus, the absorbent batt may comprise cellulosic material such as an air-formed batt of wood pulp fibers, commonly known as "fluff"; a batt of melt blown synthetic fibers, such as macrofibers or microfibers, of polypropylene, polyethylene, polyester and the like; a bonded carded web of synthetic or cellulosic fibrous materials; a composite of melt blown fibers, such as macrofibers or microfibers of polypropylene, polyethylene polyester or the like mixed with pulp fibers; or a blend of fluff with staple textile fibers such as rayon and the like. Preferably the batt should contain a high hardwood pulp content since this increases the fine microfiber pore structure in meltblown and similar batts and therefore improves absorbency and liquid retention. The batt may comprise one or more layers or combinations of the foregoing materials.

Preferably, the batt may include compounds added to increase its absorbency. The material selected for the absorbent batt most usefully has an absorbent capacity in the range of about 30 to 550 grams of synthetic urine retained at $0.69 \cdot 10^4$ Pa. For a disposable training pant intended for infant use after the diaper stage, the absorbent capacity of the batt is preferably in the range of about 350 to 500 grams of synthetic urine retained at $0.69 \cdot 10^4$ Pa. Furthermore, it is desirable that the underpant 10 not present a diaper appearance and the absorbent batt therefore most usefully comprises a low bulk, high absorbency material. Preferably the batt will contain a three-dimensional dispersion of particles of water insoluble hydrocolloid polymer. The superabsorbent is preferably dispersed uniformly in a three-dimensional configuration within the voids of the fibrous batt but in the region which is closest to the outer fabric cover, so that liquids contacting the batt penetrate freely within the batt to superabsorbent particles farthest from the skin of the wearer. There are several types of superabsorbent materials that are commercially available:

a. Grafted starch
b. Starch polyacrylic acid grafted copolymer
c. Grafted cellulose - (CMC) carboxy - methyl cellulose
d. Modified PVA (polyvinyl Alcohols), and preferably
e. Polyacrylic acid salts that are cross-linked to form absorbent polymers such as Water-Loch J-500 (Acquakeep OSH) produced by Seitetsu in Japan.

Preferably the tensile strength of the absorbent batt should be from about $2.41 \cdot 10^4$ Pa to about $6.9 \cdot 10^4$ Pa. Also preferably, the burst strength of the absorbent batt should range from about $250 \cdot 10^{-7}$ J/cm$^2$ to about $550 \cdot 10^{-7}$ J/cm$^2$ dry and from about $300 \cdot 10^{-7}$ J/cm$^2$ to about $500 \cdot 10^{-7}$ J/cm$^2$ wet.

Preferably the absorbent batt is formed having an outwardly bulging crotch portion. By contouring the batt in a manner providing the bulge in the crotch, the undergarment has a significantly greater amount of absorbent material in the crotch portion for improved protection from leakage in an area where it is most needed. In addition, advantageously decreasing the amount and thickness of the absorbent material upon approaching the periphery of the absorbent batt and the undergarment, i.e. in the longitudinal or end edges 54 and 53, provides increased discreetness for the wearer. The undergarment will not have a tendency to stick out during use. Additionally, it is preferable that the absorbent batt be shaped such that when applied to the outer cover the absorbent batt should be absent in the ear portion, i.e., the portions of the outer cover lying outside the longitudinal line extending from the innermost portion of the curved leg opening, since the undergarment does not require absorbency in this region and absence of the absorbent material provides for a more discreet fit on the wearer and does not stick out during use.

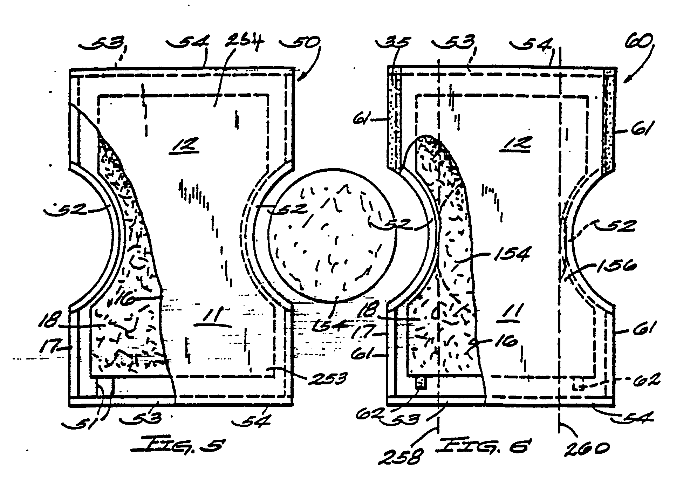

Preferably, the undergarment is formed such that the traverse measurement of the crotch section of the absorbent batt is less than about 76.2 mm. More preferably the traverse measurement of the overall diaper is less than about 101.6 mm. The thickness in the bulge portion of the absorbent batt in the crotch portion is preferably about 1.5 to 5 times the thickness of the absorbent material located in the front and rear portions of the absorbent batt. Preferably the thickness of the absorbent batt in the crotch portion is about 2 times the thickness of the absorbent material in the front and rear portions. Preferably during the formation of the absorbent batt a web of absorbent is cut traversely with a generally elliptically shaped cutout being made in the leg area. This leg area ellipse is folded over onto the top of the crotch portion. The ellipse is shown in Fig. 5 as 154; the ellipse is then placed atop the absorbent batt in Fig. 6 in crotch region 156. Preferably the traverse width of the absorbent material in the crotch portion is 1/4 of the sum of the widest traverse measurements of the front and rear portions. Forming an absorbent batt in this configuration eliminates the need for a waste of the absorbent material while unexpectedly forming an absorbent batt that provides additional absorbency in the needed areas and proper sizing about the waist.

Preferably the leg area ellipse is sized such that it covers at least 30 percent, more preferably 50 percent of the absorbent batt and is biased in size toward the front of the training pant.

In accordance with a highly preferred aspect of this invention, the outer cover 17 performs the dual functionality of providing moisture imperviousness and also providing a textile feel and look for the underpant 10. For this purpose, referring now to Fig. 3, the outer cover 17 comprises a two-layer composite material having an inner layer 30 and an outer layer 31. The inner layer 30 is a layer or stratum of plastic material which provides a moisture barrier or liquid imperviousness. Suitable polymer materials for the inner layer 30 are polyolefin polymers such as polyethylene or polypropylene; polyolefin copolymers such as ethylene vinyl acetate, ethylene methyl acrylate or ethylene ethyl acrylate; polyvinyl chloride; nylon; or other thermoplastic materials capable of providing liquid imperviousness. The inner layer 30 of the outer cover should provide a sufficient degree of liquid imperviousness to prevent or preclude waste fluids from striking through or penetrating through the outer cover. The outer layer 32 of the cover 17 most preferably consists of a layer of non-woven fibrous material. Materials suitable for the outer layer 31 include a spun-bonded non-woven web of synthetic fibers such as polypropylene, polyethylene or polyester fibers; a non-woven web of cellulosic fibers, textile fibers such as rayon fibers, cotton and the like, or a blend of cellulosic and textile fibers; a spun-bonded non-woven web of synthetic fibers such as polypropylene, polyethylene or polyester fibers mixed with cellulosic, pulp fibers or textile fibers; or melt blown thermoplastic fibers, such as macrofibers or microfibers, of polypropylene, polyethylene, polyester or other thermoplastic materials or mixtures of such thermoplastic macrofibers or microfibers with cellulosic, pulp or textile fibers.

The inner layer 30 and outer layer 31 of the cover 17 are advantageously bonded to one another, by any means appropriate for the specific materials selected for the two layers. The two layers can be laminated using heat or pressure or both heat and pressure. The two layers can also be bonded with adhesive, heat sealing or ultrasonic sealing. In addition, thermoplastic polymeric material of the inner layer 30 can be extrusion coated onto the non-woven outer layer 31. If desired, the textile effect of the non-woven outer layer 31 can be further enhanced by various embossing patterns.

Fig. 3 illustrates one form of side seams 13 for the underpant 10 that is made by seaming together exterior contacting side edge portions of the outer layer 31 of the outer cover 17 of the front and rear panels. This provides a narrow fin seam, which can be made relatively narrow such as about 4.76 to 12.7 mm wide so as to minimize the amount of seam which is visible. The seams 13 between the outwardly contacting side edge portions of the outer cover can be formed by any suitable means well known in the art appropriate to the specified material employed for the outer layer 31 of the cover; thus, sonic sealing, heat sealing, adhesive bonding, and the like are appropriate techniques. Fig. 4 illustrates an alternate construction for the side seams 13 wherein side edge portions of the garment are overlapped and joined together with an adhesive element 35 coated on both of its opposed surfaces with layers 36 and 37 of pressure sensitive adhesive. In this alternate embodiment, a side edge portion of the bodyside inner liner 16 of the rear panel 12 is bonded to a side edge portion of the outer layer 31 of the outer cover of the front panel 11 of the underpant 10. Sonic sealing and heat sealing techniques also can be used to bond the over-

lapped portions as shown in Fig. 4. Other side seam constructions effective to form a garment having closed sides can be employed to form the underpant 10, and sewn side seams may be of interest such as for some types of adult underpants. Another embodiment includes an exterior seam, not shown.

An especially useful construction for the side seams 13 is a manually tearable or tearaway seam. This can be obtained by bonding the contacting side edge portions along a narrow bond within the side seam portions. A bond strength of about 78 grams/mm of seam length (as measured on an appropriate instrument such as an Instron tensile tester) is suitable for providing a tearaway seam but which is also strong enough to hold the garment together. One way to make a seam of this type is to bond the contacting side portions by suitably controlled sonic sealing along a narrow bond portion that is about 3.17 mm wide. A tearaway seam is a highly advantageous and preferred feature because a parent can manually tear the side seams apart in order to remove the underpant from a child; this is particularly helpful when the underpant is quite soiled and removal in the normal fashion would be messy. A manually tearaway seam can be with both the inturned fin style seams illustrated in Fig. 3 or the overlapped seam of Fig. 4. Another important consideration in forming a strong bond which provides for a tearaway feature includes selection of a suitable material for the cover. Propropylene, ethylene vinyl acrylate, ethylene ethyl acrylate and ethylene methyl acrylate are preferred. Polyethylene is not preferred but ethylene methyl acrylate copolymers of ethylene may be used.

The side seams 13 in Fig. 3 are shown as incorporating another useful structural feature. The inturned contacting side edge portions of the garment are joined together along a narrow bond portion 25 that is spaced from the free ends 26 of the side edge portions. This provides a side seam having a flap portion 27 inside the garment along which the side edge portions are not bonded together so as to be free of one another. Any bond portion, such as formed by sonic sealing, heat sealing or adhesive bonding, will be relatively stiff. The flap portion 27 acts as a cushion between a person's body and the stiff bond portion 25, thereby enhancing the comfort or wearability of the underpant 10. It has been found that a bond portion about 1.58 mm to 3.17 mm wide and a flap portion about 3.17 mm to 9.52 mm wide are suitable for this purpose and provide a fin seam that will not irritate an infant or adult wearing the underpant 10.

The underpant 10 can be expeditiously manufactured from a blank cut to a suitable configuration. An appropriate blank 50 is illustrated in Fig. 5. A sheet of material for the outer cover 17 is cut to an hourglass configuration having arcuate cutouts defining the leg openings of the garment. Absorbent batt 18, also cut to an hourglass configuration with arcuate leg cutouts, is placed on

top of the outer cover in the desired position, and may be secured thereto by spaced parallel glue lines 51. Preferably the batt is shaped such that the extensions of the batt in the areas outside longitudinal lines 258 and 260 in Fig. 6 extending from the traverse edge of the inside crotch portion is limited. An arcuate elastic means 52 is positioned around each leg cutout and preferably, as explained below, positioned along the outer edge of the cover 17 along the cutout portion. Linear elastic means 53 are positioned along each end 54 of the blank, which will form the elastic means for the waist portion of the finished garment. As indicated in Fig. 5, the elastic means 52 are positioned closely adjacent the nearby edge of the absorbent batt so as to provide a form fitting panty type of garment instead of being spaced from the batt to have a web of material between the elastic means and the batt as is common with some disposable diapers. Next, a sheet of bodyside inner liner 16, also cut to an hourglass configuration, is placed over the assembly of the outer cover and the batt. Both the liner 16 and cover 17 have superimposed marginal portions which project beyond the margin of the batt 18, and the liner and cover may be joined together with elastics 52 and 53 within the superimposed marginal portions. After being fully assembled, the blank 50 is folded along its central transverse area and the sides of the front and rear panels are seamed together as illustrated in Fig. 3 to form the finished underpant 10.

Fig. 6 illustrates a blank 60 suitable for producing underpant 10 having side seams of the type illustrated in Fig. 4. Most elements of the blank 60 are the same as those of the blank 50 and the corresponding elements are therefore numbered with the same reference numerals. Blank 60 includes an adhesive element 35 of two-side coated pressure sensitive adhesive tape along each side edge portion 61 of the rear panel 12. Thus (see especially Fig. 4) adhesive layer 36 of each element 35 is adhered to a side marginal portion of the outer cover of the rear panel 12. To produce the underpant 10 from the blank 60, the blank is folded along its transverse medial portion and the side edge portions 61 of the front panel are joined together to the layer 37 of pressure sensitive adhesive of each element 35 in the manner shown in Fig. 4. The blank 60 also shows an alternate form of securing the absorbent batt 18 by use of two side pressure sensitive adhesive elements 62 between the batt and outer cover 17.

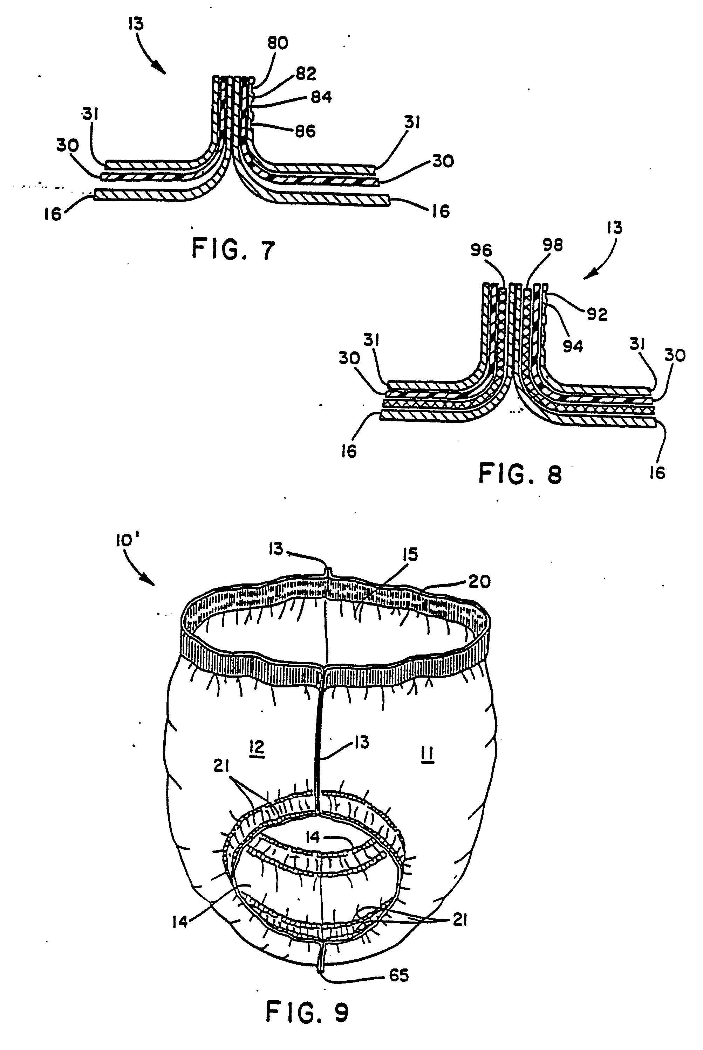

Fig. 7 is an enlarged view of the cross-sectional view of seam 13 in Fig. 3. The seam is composed of a six-layered structure of the outer fabric layer 31, the inner impermeable layer 30 of the cover sheet, as well as the permeable layer 16 forming the liner of the garment. Therefore, the seal 13 has six layers, all of which must be sealed together to form a small unobtrusive bond. This is done by sealing with a series of lines 80 that form the pressured areas 80 and raised lined areas 82. The sealing is accomplished within a preferred width of about 3.17 mm with a plurality of sealing lines being formed within this space. As illustrated, there are three sealing lines 80, 84, and 86 within the seal 13. Surprisingly, it has been found that the multi-line sealing carried out with ultrasonic sealing is stronger when a series of narrow lines are formed rather than a larger continuous sealed area.

The series of seal lines may be formed by any desired method. Typical of such methods are adhesive and heat sealing. A particularly preferred system for the invention is found to be ultrasonic sealing. It has been found that an ultrasonic sealing anvil having a plurality of lines closely spaced together such that four sealing lines may be fit within the preferred space of between 3.17 mm and 4.76 mm is particularly desirable. The sealing lines may be discontinuous, forming dashed lines.

In formation of the lines an ultrasonic apparatus suitable is a Branson 851 Model ultrasonic Sealing Unit and with a preferred anvil of 3.17 mm width and 152.4 mm length having engraved thereon a line pattern of about 0.45 mm wide lands and valleys about 0.35 mm wide forming four lines in a total width of about 3.17 mm. This anvil is suitably applied with $483 \cdot 10^4 - 2340 \cdot 10^4$ Pa force for .05 - 2.0 seconds to the composite forming the side seal of the garment to laminate the front and back portions together. The preferred anvil force is about $552 \cdot 10^4 - 828 \cdot 10^4$ Pa force for about 0.3 seconds.

Illustrated in Fig. 8 is a cross-section of the garment 10 taken on section line 6 of Fig. 1 such that the cross-section of the seam 13 is taken at the waist elastic. Surprisingly it has been found that the ultrasonic sealing forming a pattern of sealed valleys 92 and raised lines 94 between the valleys even seals in this area where the front and back waist elastic 96 and 98 are present in the seam.

Fig. 9 illustrates an alternate system for construction of the underpant of the present invention wherein the underpant 10' includes a separate front panel 11 and rear panel 12 that are joined together along central crotch seam 65. The remaining elements of the underpant 10' are the same as in underpant 10 and the common elements are identified with the corresponding reference numerals employed in Fig. 1. Fig. 9 represents an alternative method for constructing the underpants of the present invention as compared to making the underpants with the folded blanks 50 of Fig. 5.

The discrete, strong, non-leaking side seal of the invention may be formed in any desired width. Generally the seam would have a width of between about 1.58 mm and about 4.76 mm. It is preferred that the seam have a total width of about 3.17 mm. Within the seam area encompassed by the lines of sealing in the seam's bonding area the sealed valley portions are generally about 25 % to about 100 % of the total. The number of lines may be any desired number greater than 1. Generally it has been found that four sealing lines in a space of about 3.17 mm with about 50 % seal area are suitable.

Any suitable elastomeric material can be employed for the elastic in the garments of the invention that exhibits at least an elongation (defined herein as $L_s - L_r / L_r$ where $L_s$ is the stretched length of an elastic element and $L_r$ is retracted length, multiplied by 100 to obtain percent elongation) in the range of 5 % to 300 % preferably in the range of 25 % to 200 %. Further along these lines, there may be some preferential vagaries in respect of the elasticity of these elastic means relative to the geometry elected by the designed. For example, within the preferred range mentioned above, it has been determined that a most preferred range for the leg elastic is from about 80 to about 110 for rope elastic and about 40 to about 70 for ribbon elastic. Also for the waist elastic the preferred range is from bout 200 to about 250 for rope elastic and about 60 to about 200 for ribbon elastic. Preferably the elastic has a spring constant of from about 5 to about 40 gms/cm and more preferably from about 15 to about 22 gms/cm.

Various commercially available materials can be used, such as natural rubber, butyl rubber or other synethetic rubber, urethane elastomeric material such as that available from B. F. Goodrich Company under the trademark TUFTANE, and elastomeric material available from the H. B. Fuller Company under the tradename FULLASTIC. The latter material (see e.g. U.S. Patent 4 418 123) is based upon thermoplastic elastomeric copolymers of the A-B-A type such as those available from shell Chemical under the trademark KRATON which have a rubbery mid-block such as butadiene or isoprene and polystyrene end blocks, and is especially useful because it is a self-adhesive material and can be applied to the layers of the garment without additional adhesive between the elastic means and the layers. The elastic means can be applied to the garment by any suitable means including adhesive bonding, heat sealing or sonic bonding, whichever is appropriate to the specific material selected for the elastic means.

Fig. 12 illustrates an alternate system for construction of the underpant of the present invention wherein the underpant 10' includes a separate front panel 11 and rear panel 12 that are joined together along central crotch seam 65. The remaining elements of the underpant 10' are the same as in underpant 10 and the common elements are identified with the corresponding reference numerals employed in Fig. 1. Fig. 12 represents an alternative method for constructing the underpants of the present invention as compared to making the underpants with the folded blanks 50 and 60 of Figs. 5 and 6.

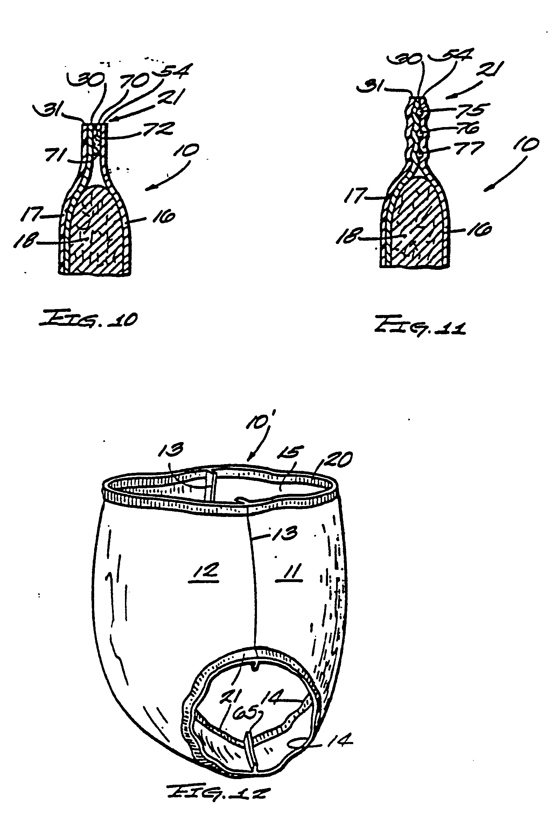

Figs. 10 and 11 illustrate particularly useful forms of elastic means for use at the leg openings and waist opening of the underpant 10. The elastic means constructions of Figs. 10 and 11 are as described and claimed in the commonly assigned co-pending patent application of Ales et al, entitled "Elastic Form-Fitting Closure Constructions for Disposable Garments," filed on January 10, 1985, U.S. Serial No. 690 348, the disclosure which is incorporated herein by reference. As shown in Fig. 10, elastic means 54 comprises a strip of elastic material 70 having opposed surfaces 71 and 72, wherein surface 71 is bonded to closely spaced bond points along an edge portion of the inner layer 30 of the outer cover 17 and surface 72 is bonded to an edge portion of the interior surface of the bodyside liner 16. Further, the outer edges of the liner 16, cover 17, and elastic strip 70 are contiguous with one another. The elastic strip 70 is bonded to the liner 16 and cover 17 along the entire area of its surfaces 71 and 72 when applied thereto in an elongated or stretched condition; upon retraction of the elastic strip 70, micro-buckling of the outer cover between bond points results in an elasticized waist or leg opening which has a smooth yet finely ribbed or pleated appearance. Fig. 10 is a sectional view of the elasticized band 21 around the leg openings of the garment 10, and the elasticized band 20 around the waist opening can be of the same structure.

The construction illustrated in Fig. 11 is similar to that of Fig. 10 except that the elastic means 53 comprises a plurality of spaced parallel strands 75, 76 and 77 of elastic material, each strand having a circular cross-section, as described and claimed in the commonly assigned co-pending application of Ales et al, entitled "Disposable Garment with Multiple Strand Elasticized Openings," U.S. Serial No. 690 349, the disclosure of which is incorporated herein by reference. The elastic means construction of Fig. 8 can be used either for the circumferential elasticized band 20 around the waist opening of the underpant or the elasticized bands 21 around the leg openings 14 of the underpant, or both; a presently preferred embodiment of the underpant 10 is to employ the elastic means construction of Fig. 10 for the circumferential band 20 about the waist opening and the elastic means construction of Fig. 11 for the elasticized band 21 of the leg openings with a good seal against leakage of fluids therethrough. The underpant 10, however, can be constructed with other types of elasticized bands at the waist opening and the leg openings, such as, for example, a strip of elastic material intermittently bonded or sewn to either the outer cover 17 or inner liner 16, or both, along the waist opening and leg openings. Preferably the elastic means about the waist and/or the legs is at least two ribbons or ropes. Also, the elastic means can be contiguous with the outer edges of the liner and cover along the elasticized openings as shown in Figs. 10 and 11, or it can be spaced inwardly thereof; in the latter instance, it is preferred that the elastic means be spaced only slightly inwardly of the edges of the garment if it is desired to avoid a gathered look about the leg and waist openings.

The tension measurement found useful was determined by stretching the leg opening to 266.7 mm for the small training pant (9 - 13 kg) and 292.1 mm for the medium training pant (14 – 18 kg), holding for five minutes and then measuring the tension in grams. A similar procedure was followed for the waist except the opening was stretched to 470 mm for the small and 495 mm for the large. These stretching distances were determined as being the average leg and waist size for the wearer of the small training pant and the medium training pant. Preferably the tension exerted by the elastic means in the leg area ranges from about 165 to about 220 grams, more preferably from about 180 to about 200 grams. The elastic means about the leg opening preferably comprises at least two elastic members where the tension on the member closer to the absorbent batt is greater than the tension on the elastic member further from the absorbent batt. More preferably the ratio of the tension on the members is from about 4 : 3 to about 3 : 2. In the waist region the preferred tension is from about 330 to about 400 grams and more preferably from about 360 to about 380 grams.

Any suitable elastomeric material can be employed for the elastic means 52 and 53 that exhibits at least an elongation (defined herein as $L_s - L_r / L_r$ where $L_s$ is the stretched length of an elastic element and $L_r$ is retracted length, multiplied by 100 to obtain percent elongation) in the range of 5 % to 300 % preferably in the range of 25 % to 200 %. Further along these lines, there may be some preferential vagaries in respect of the elasticity of these elastic means relative to the geometry elected by the designer. For example, within the preferred range mentioned above, it has been determined that a most preferred range for the leg elastic is from about 80 to about 110 for rope elastic and about 40 to about 70 for ribbon elastic. Also for the waist elastic the preferred range is from about 200 to about 250 for rope elastic and about 60 to about 200 for ribbon elastic. Preferably the elastic has a spring constant of from about 5 to about 40 gms/cm and more preferably from about 15 to about 22 gms/cm.

Various commercially available materials can be used, such as natural rubber, butyl rubber or other synthetic rubber, urethane elastomeric material such as that available from B. F. Goodrich Company under the trademark TUFTANE, and elastomeric material available from the H. B. Fuller Company under the tradename FULLASTIC. The latter material (see e.g. U.S. Patent 4 418 123) is based upon thermoplastic elastomeric copolymers of the A-B-A type such as those available from Shell Chemical under the trademark KRATON which have a rubbery midblock such as butadiene or isoprene and polystyrene end blocks, and is especially useful because it is a self-adhesive material and can be applied to the layers of the garment without additional adhesive between the elastic means and the layers. The elastic means can be applied to the garment by any suitable means including adhesive bonding, heat sealing or sonic bonding, whichever is appropriate to the specific material selected for the elastic means.

Adhesion of the elastics 20 and 21 to the liner 16 and to the cover 17 is improved by addition of a dissimilar polymer to the liner/cover material. This is illustrated by addition of polypropylene to an ethylene methyl acrylate cover. It has been convenient to add the dissimilar polymer by addition of a yellow colorant Ampacet containing a yellow pigment and a polypropylene binder. This addition has been found particularly effective in EMA when a Fullastic self adhering elastic is used in the training pant.

Preferably, the front and back of the training pant are designated as such so as to permit the wearer to properly dress himself. This may be suitably accomplished by a label on the interior of the rear portion of the training pant.

### Example

A disposable panty 10 as illustrated in Fig. 1 was constructed in a size suitable for use as a child's training panty with an elasticized waist opening as shown in Fig. 10, elasticized leg openings as shown in Fig. 11 and tearaway side seams. The material of the outer cover, or exterior panel, of the disposable underpant was a two-layer composite web having an outer layer of non-woven polypropylene fibers and an inner layer of ethylene methyl acrylate extrusion coated onto the non-woven fibrous outer layer. The panty had an interior panel comprising a bodyside liner of spun bonded polypropylene fibers. An absorbent batt of a composite of polypropylene microfibers and cellulosic fibers was sandwiched between the exterior panel and the interior panel.

The exterior seams were sealed ultrasonically with a series of 4 lines within a side seal of about 3.17 mm. Testing of the panty established that it combined the features of liquid imperviousness due to the plastic inner layer of the outer cover and a cloth-like appearance because of the fibrous outer layer of the outer cover. The sealing of the side seal seams was strong and complete and did not detract from the panty-like appearance of the garment. A useful disposable training pant was thereby provided that is expected to be well-received by parents and of a type that will aid and encourage children going through the toilet training stage.

There has thus been described a disposable underpant including an outer cover constructed of two layers of different materials wherein the inner layer is a plastic material capable of providing the desired degree of moisture imperviousness and the outer layer is a non-woven fibrous material capable of presenting a cloth-like or textile appearance and feel to the underpant. The garment further has an exterior side seal seam that is capable of holding the garment together without being obtrusive. It is believed that the new underpant herein described provides a construction that is an improvement over similar garments of the prior art and that it may therefore extend the use of disposable underpants, particularly as a training pant for a child who is ready to graduate from the diaper stage. The texture and feel, as well as the fit and absorbent capabilities, of the present underpant provide a disposable undergarment that can function to contain waste fluids and at the same time present a cloth-like appearance which could encourage an infant to discontinue use of diapers. The foregoing disposable underpant can be more absorbent than a cloth training pant and does not require an additional protective rubber or plastic covering pant. Because the present garment has elasticized waist and leg openings, a young child of an appropriate age can readily raise and lower the underpant and thereby become accustomed to using the toilet without being dependent upon the aid of a parent. The neat tailored look of the present underpant should be of aid in promoting the use of the training pant in lieu of a diaper. Other important potential uses for the underpant herein described and claimed are an adult menstrual garment or an adult incontinence garment. The same factors come into play in these end uses, since a cloth-like disposable underpant having an attractive tailored look is provided that should be particularly attractive for an adult.

The outside seal seam of the invention is particularly desirable when composite materials containing spunbonded material such as polypropylene or polyethylene are present. In the illustration set forth above the sealing system was satisfactory for sealing even when eight layers including adhesive layers were present. It is particularly desirable for a multi-layer sealing of thermoplastic materials. The seal of the invention is stronger than a single large seal and additionally the line structure does not detract extensively from the clothlike outer surface of the spun-bonded surface on the garment illustrated.

While the invention has been specifically described with respect to training pants, other types of garments may also be formed by the sealing system of the invention utilizing an ultrasonically-sealed narrow seam having a plurality of lines of sealing. Other garments that the seal could be used for are training pants that have absorbent inserts rather than the disposable pants illustrated. Other garments to benefit by the side seam of the invention could be disposable bathing suits. The invention is also suitable for hospital garments such as gowns, as well as hospital underwear. Further, the invention could be utilized in formation of cloth-like bags, and particularly is suitable for formation of cloth-like disposable bags as it is easier to make them using the system of the invention as the garment or bag does not need to be turned inside out after formation.

The present invention has been described hereinabove by reference to several specific embodiments, but it is expected that those skilled in the art of manufacturing disposable garments will be able to devise modifications of the exemplary embodiments and it is intended that the appended claims encompass any such obvious modifications which are within the true spirit and scope of the present invention.

Claims

1. A disposable underpant of the type having a liquid-pervious inner bodyside liner (16) and a liquid-impervious outer cover (17) that define a front panel (11) and a rear panel (12), and an absorbent batt (18) between the liner and the cover, comprising, in combination:

(1) side seams (13) joining together part of marginal side portions of the front and rear panels (11, 12) to define a three-dimensional underpant having a pair of leg openings (14) and a waist opening (15);
(2) first elastic means (52) extending about one leg opening, second elastic means (52) extending about the other leg opening, and third elastic means (54) extending about the waist opening; characterized in that:
the outer cover (14) consists of an inner layer (30) of liquid-impervious plastic material and an outer layer (31) of non-woven fibrous material, so that the inner layer (30) faces the absorbent batt (18) and the outer layer (31) is the exterior surface of the disposable underpant.

2. The undergarment of Claim 1 wherein the side seams (13) comprise at least one sealed exterior seam, at least one said seam being sealed with two narrow lines (80, 84) and the total seam being less than about 4.76 mm side.

3. The underpant of Claim 1 wherein the side seams (13) join together inturned contacting side edge portions of the front and rear panels arranged inside the underpant.

4. The underpant of Claim 3 wherein the side seams (13) comprise a bond portion (25) in the range of 1.58 mm to 3.17 mm wide between the intuned side edge portions and a flap portion in the range of 3.17 mm to 9.52 mm wide between the bond portion (25) and the free ends (26) of the inturned side edge portions along which the inturned side edge portions are free of one another.

5. The underpant of Claim 1 wherein the side seams (13) join together overlapped contacting side edge portions of the front and rear panels (11, 12).

6. The underpant of Claim 1, 2, 3, 4 or 5, wherein the side seams (13) include manually tearaway bonds between the front and rear panels.

7. The underpant of Claim 1 wherein said seams (13) are sealed across the waist elastic (53).

8. The underpant of Claim 1 wherein said sealing is ultrasonic.

9. The underpant of Claim 1 wherein said seam (13) is about 3.17 mm wide and bonded on about 50 % of said total seam area.

10. The underpant of Claim 1 wherein said seam (13) is also at an elastic leg (52) and elastic waist (53).

11. The underpant of Claim 1 wherein said at least one seam (13) comprises an ultrasonically-sealed seam at each side of said garment.

12. The underpant of Claim 1 wherein each seam comprises four narrow sealing lines (92, 94 etc.).

13. The underpant of Claim 1 wherein each seam (13) is about 3.17 mm wide and comprises four sealing lines (92, 94, etc.).

14. The underpant of Claim 2, wherein said lines are discontinuous.

15. The underpant of Claim 1, 2, 3, 4 or 5, wherein the inner layer (30) of the outer cover (17) is a layer of thermoplastic material extrusion coated into the outer layer of the outer cover.

16. The undergarment of Claim 15 wherein said absorbent batt (18) is formed having a front portion, a rear portion, and a crotch portion corresponding to said outside cover fabric.

17. The undergarment of Claim 15 wherein the traverse measurement of the crotch portion of said absorbent batt (18) is less than about 76 mm.

18. The undergarment of Claim 15 wherein the traverse width of the absorbent material (18) in the crotch portion is 1/4 of the sum of the widest traverse measurements of the front and back portions.

19. The underpant of Claim 1 wherein the thickness of the absorbent batt in the crotch portion is from about 1.5 to about 5 times the thickness of the absorbent batt in said front and rear panels.

20. The underpant of Claim 1 wherein the absorbent batt (18) is outwardly bulging in the crotch portion.

21. The underpant of Claim 1 wherein said absorbent batt (18) is attached to said outer cover.

22. The underpant of Claim 1 wherein said absorbent batt (18) comprises a superabsorbent.

23. The underpant of Claim 22 wherein said superabsorbent is confined to a pre-determined region with said absorbent batt (18).

24. The underpant of Claim 22 wherein said superabsorbent is disposed in the lower region of said batt (18) immediately adjacent to said outer cover.

25. The underpant of Claim 1 wherein said first and second elastic means (52) comprises at least two elastic members (76, 77) wherein the tension on the member closest to said absorbent batt is greater than the tension on the member furthest from said absorbent batt.

26. The underpant of Claim 25 wherein the ratio of the tension on said closest member (77) to said furthest member (76) is from about 4 : 3 to 3 : 2.

27. The underpant of Claim 1 wherein the tension on said first and second elastic means (52) ranges from about 165 to about 220 grams.

28. The underpant of Claim 1 wherein the tension on the third elastic means (53) ranges from about 330 to about 450 grams.

29. The underpant of Claim 1 wherein said absorbent batt (18) is substantially absent from the front and rear panels traversely extending beyond a longitudinal line (258, 260) extending from outside traverse edges of said crotch portion.

30. The underpant of Claim 1 wherein said outer cover (17) comprises stretchable material.

31. The underpant of Claim 1 wherein said absorbent (18) batt comprises a material having a dry tensile strength ranging from about $250 \cdot 10^{-7}$ $J/cm^2$ to about $550 \cdot 10^{-7}$ $J/cm^2$.

**Patentansprüche**

1. Wegwerfbare Unterhose, enthaltend eine flüssigkeitsdurchlässige, innere, körperseitige Lage (16) und eine flüssigkeitsundurchlässige äußere Decke (17), die ein Vorderteil (11) und ein Hinterteil (12) bilden, und eine absorbierende Einlage (18) zwischen der Lage und der Decke, enthaltend in Kombination:

(1) Seitennähte (13), die einen Teil der Randseitenabschnitte der Vorder- und Hinterteile (11, 12) miteinander verbinden, um eine dreidimensionale Unterhose zu bilden, die zwei Beinöffnungen (14) und eine Taillenöffnung (15) hat;
(2) eine erste elastische Einrichtung (52), die sich um eine Beinöffnung herumerstreckt, eine zweite elastische Einrichtung (52), die sich um die andere Beinöffnung herumerstreckt, und eine dritte elastische Einrichtung (54), die sich um die Taillenöffnung herumerstreckt; *dadurch gekennzeichnet, daß*
die äußere Decke (17) aus einer inneren Lage (30) aus flüssigkeitsundurchlässigem Plastikmaterial und einer äußeren Lage (31) aus ungewebtem Fasermaterial besteht, so daß die innere Lage (30) der absorbierenden Einlage (18) gegenüberliegt und die äußere Lage (31) die Außenseite der wegwerfbaren Unterhose ist.

2. Unterbekleidungsstück nach Anspruch 1, bei dem die Seitennähte (13) wenigstens einen versiegelten äußeren Saum enthalten, wobei dieser wenigstens eine Saum an zwei schmalen Linien (80, 84) abgedichtet ist und der gesamte Saum weniger als etwa 4,75 mm breit ist.

3. Unterhose nach Anspruch 1, bei der die Seitennähte (13) sich nach innen gewendet vereinigen, Seitenrandbereiche der Vorder- und Hinterteile berührend, die an der Innenseite der Unterhose angeordnet sind.

4. Unterhose nach Anspruch 3, bei der die Seitennähte (13) einen Verbindungsabschnitt (25) im Bereich von 1,58 mm bis 3,17 mm Breite zwischen den nach innen gewendeten Seitenrandbereichen und einen Klappenabschnitt im Bereich von 3,17 mm bis 9,52 mm Breite zwischen dem Verbindungsabschnitt (25) und den freien Enden (20) der nach innen gewendeten Seitenrandbereiche aufweisen, längs denen die nach innen gewendeten Seitenrandbereiche voneinander frei sind.

5. Unterhose nach Anspruch 1, bei der die Seitennähte (13) sich überlappend vereinigen, Seitenrandabschnitte der Vorder- und Hinterteile (11, 12) berührend.

6. Unterhose nach Anspruch 1, 2, 3, 4 oder 5, bei der die Seitennähte (13) manuell wegreißbare Verbindungen zwischen den Vorder- und Hinterteilen enthalten.

7. Unterhose nach Anspruch 1, bei der die Nähte (13) über die elastische Einrichtung (53) an der Hüfte hinweg versiegelt sind.

8. Unterhose nach Anspruch 1, bei der die Versiegelung eine Ultraschallversiegelung ist.

9. Unterhose nach Anspruch 1, bei der die Naht (13) etwa 3,17 mm breit ist und aus etwa 50 % der gesamten Nahtfläche angeheftet ist.

10. Unterhose nach Anspruch 1, bei der die genannte Naht (13) auch an dem elastischen Bein (52) und an der elastischen Hüfte (53) ist.

11. Unterhose nach Anspruch 1, bei der die genannte wenigstens eine Naht (13) eine ultraschallverschweißte Naht an jeder Seite des Bekleidungsstücks aufweist.

12. Unterhose nach Anspruch 1, bei der jede Naht vier schmale Versiegelungslinien (92, 94 usw.) enthält.

13. Unterhose nach Anspruch 1, bei der jede Naht (13) etwa 3,17 mm breit ist und vier Versiegelungslinien (92, 94 usw.) enthält.

14. Unterhose nach Anspruch 2, bei der die genannten Linien diskontinuierlich sind.

15. Unterhose nach Anspruch 1, 2, 3, 4 oder 5, bei der die innere Lage (30) der äußeren Decke (17) eine Lage aus thermoplastischem Material ist, das auf die äußere Lage der äußeren Decke extrusionsbeschichtet ist.

16. Unterbekleidungsstück nach Anspruch 15, bei dem die absorbierende Einlage (18) so ausgebildet ist, daß sie einen Vorderabschnitt, einen Hinterabschnitt und einen Schrittabschnitt entsprechend dem äußeren Deckstoff hat.

17. Unterbekleidungsstück nach Anspruch 15, bei dem das Quermaß des Schrittabschnitts der absorbierenden Einlage (18) kleiner als 76 mm ist.

18. Unterbekleidungsstück nach Anspruch 15, bei dem die Querbreite des absorbierenden Materials (18) im Schrittabschnitt 1/4 der Summe der breitesten Querabmessungen der Vorder- und Hinterabschnitte ist.

19. Unterbekleidungsstück nach Anspruch 1, bei dem die Dicke der absorbierenden Einlage (18) im Schrittabschnitt das etwa 1,5- bis 5-fache der Dicke der absorbierenden Einlage in den Vorder- und Hinterteilen hat.

20. Unterhose nach Anspruch 1, bei der die absorbierende Einlage (18) im Schrittbereich nach außen gewölbt ist.

21. Unterhose nach Anspruch 1, bei der die absorbierende Einlage (18) an der äußeren Decke befestigt ist.

22. Unterhose nach Anspruch 1, bei der die absorbierende Einlage (18) ein Superabsorbens enthält.

23. Unterhose nach Anspruch 22, bei der das Superabsorbens auf einen vorbestimmten Bereich mit der absorbierenden Einlage (18) beschränkt ist.

24. Unterhose nach Anspruch 22, bei der das Superabsorbens im unteren Bereich der Einlage (18) unmittelbar benachbart der äußeren Decke angeordnet ist.

25. Unterhose nach Anspruch 1, bei der die ersten und zweiten elastischen Einrichtungen (52) wenigstens zwei elastische Elemente (76, 77) enthalten, wobei die Spannung an dem Element, das der absorbierenden Einlage am nächsten liegt, größer ist, als die Spannung an dem Element, das von der absorbierenden Einlage am weitesten entfernt ist.

26. Unterhose nach Anspruch 25, bei der das Verhältnis der Spannung an dem naheliegendsten Element (77) zu der am entferntesten liegenden Element (76) zwischen etwa 4 : 3 und etwa 3 : 2 liegt.

27. Unterhose nach Anspruch 1, bei der die Spannung an den ersten und zweiten elastischen Einrichtungen (52) im Bereich zwischen etwa 165 und etwa 220 Gramm liegt.

28. Unterhose nach Anspruch 1, bei der die Spannung an der dritten elastischen Einrichtung (53) zwischen etwa 330 und etwa 450 Gramm liegt.

29. Unterhose nach Anspruch 1, bei der die absorbierende Einlage (18) an den Vorder- und Hinterteilen an Stellen, die sich quer über eine Längslinie (258, 260) hinauserstrecken, fehlt, welche Längslinie sich von äußeren Querrändern zum Zwickelabschnitt erstreckt.

30. Unterhose nach Anspruch 1, bei der die äußere Decke (17) aus einem dehnbaren Material besteht.

31. Unterhose nach Anspruch 1, bei der die absorbierende Einlage (18) aus einem Material besteht, das eine Trockendehnungsfestigkeit im Bereich zwischen etwa 250 x 10$^{-7}$ J/cm$^2$ und etwa 550 x 10$^{-7}$ J/cm$^2$ hat.

**Revendications**

1. Culotte à jeter du type ayant une doublure intérieure (16), côté corporel, perméable aux liquides, une couverture extérieure (17) imperméable aux liquides qui définissent un panneau avant (11) et un panneau arrière (12), et un matelas absorbant (18) compris entre la doublure et la converture, la culotte comprenant, en combinaison:

(1) des jointures latérales (13) réunissant ensemble des parties de portions latérales marginales des panneaux avant et arrière (11, 12) pour définir une culotte tridimensionnelle ayant une paire d'ouvertures de jambes (I4) et une ouverture de taille (15);
(2) des premiers moyens élastiques (52) s'étendant autour d'une ouverture de jambe, des seconds moyens élastiques (52) s'étendant autour de l'autre ouverture de jambe et des troisièmes moyens élastiques (54) s'étendant autour de l'ouverture de taille; caractérisée en en ce que: la couverture extérieure (17) est formée d'une couche interne (30) de matière plastique imperméable aux liquide set d'une couche externe (31) de matière fibreuse non tissée de telle sorte que la couche interne (30) est en vis-à-vis du matelas absorbant (18) et que la couche externe (31) constitue la surface extérieure de la culotte à jeter.

2. Sous-vêtement selon la revendication 1, dans lequel les jointures latérales (13) comprennent au moins une jointure extérieure scellée, l'une au moins desdites jointures étant scellée par deux lignes étroites (80, 84) et la jointure totale ayant moins d'environ 4,76 mm de côté.

3. Culotte selon la revendication 1, dans laquelle les jointures latérales (13) réunissent ensemble les parties de bordures latérales en contact des panneaux avant et arrière, tournées vers l'intérieur de la culotte.

4. Culotte selon la revendication 3, dans laquelle les jointures latérales (13) comprennent une partie de liaison (25) d'une largeur allant de 1,58 mm à 3,17 mm entre les parties de bordures latérales tournées vers l'intérieur et une partie formant volet ayant une largeur allant de 3,17 mm à 9,52 mm entre la partie de liaison (25) et les extrémités libres (20) des parties de bordures latérales tournées vers l'intérieur au long desquelles les parties de bordures latérales tournées vers l'intérieur sont indépendantes l'une de l'autre.

5. Culotte selon la revendication 1, dans laquelle les jointures latérales (13) réunissent ensemble des parties de bordures latérales en contact de chevauchement des panneaux avant et arrière (11, 12).

6. Culotte selon la revendication 1, 2, 3, 4 ou 5, dans laquelle les jointures latérales (13) comprennent des liaisons arrachables manuellement entre les panneaux avant et arrière.

7. Culotte selon la revendication 1, dans laquelle lesdites jointures (13) sont scellées à cheval sur l'élastique de taille (53).

8. Culotte selon la revendication 1, dans laquelle ledit scellement est fait par ultra-sons.

9. Culotte selon la revendication 1, dans laquelle ladite jointure (13) a une largeur d'environ 3,17 mm et en ce qu'elle est liée sur approximativement 50 % de sa surface totale.

10. Culotte selon la revendication 1, dans laquelle ladite jointure (13) existe également au niveau d'une jambe élastique (52) et d'une taille élastique (53).

11. Culotte selon la revendication 1, dans laquelle ladite jointure (13) est formée d'une jointure scellée par ultra-sons de chaque côté de ladite culotte.

12. Culotte selon la revendication 1, dans laquelle chaque jointure est formée de quatre lignes de scellement étroites (92, 94, etc.).

13. Culotte selon la revendication 1, dans laquelle chaque jointure (13) a une largeur d'environ 3,17 mm et en ce qu'elle comprend quatre lignes de scellement (92, 94, etc.).

14. Culotte selon la revendication 2, dans laquelle lesdites lignes sont discontinues.

15. Culotte selon la revendication 1, 2, 3, 4 ou 5, dans laquelle la couche interne (30) de la couverture extérieure (17) est une couche de matériau thermoplastique appliquée par extrusion dans la couche externe de la couverture extérieure.

16. Culotte selon la revendication 15, dans laquelle ledit matelas absorbant (18) comporte une partie avant, une partie arrière et une partie d'entre-jambes correspondant audit textile de couverture extérieure.

17. Culotte selon la revendication 15, dans laquelle la dimension transversale de la partie d'entre-jambes dudit matelas absorbant (18) est inférieure à 76 mm.

18. Culotte selon la revendication 1, dans laquelle la largeur transversale du matériau absorbant (18) dans la partie d'entre-jambes est égale au quart de la somme des mesures transversales les plus larges des parties avant et arrière.

19. Culotte selon la revendication 1, dans laquelle l'épaisseur dudit matelas absorbant (18) dans la partie d'entre-jambes est comprise entre environ 1,5 et environ 5 fois l'épaisseur du matelas absorbant dans lesdits panneaux avant et arrière.

20. Culotte selon la revendication 1, dans laquelle le matelas absorbant (18) est bombé vers l'extérieur dans la portion d'entre-jambes.

21. Culotte selon la revendication 1, dans laquelle ledit matelas absorbant (18) est fixé à ladite couverture extérieure.

22. Culotte selon la revendication 1, dans laquelle ledit matelas absorbant (18) renferme un superabsorbant.

23. Culotte selon la revendication 22, dans laquelle ledit superabsorbant est limité à une région prédéterminée dudit matelas absorbant (18).

24. Culotte selon la revendication 22, dans laquelle ledit superabsorbant est disposé dans la région inférieure dudit matelas (18), immédiatement au voisinage de la couverture extérieure.

25. Culotte selon la revendication 1, dans laquelle lesdits premiers et lesdits seconds moyens élastiques (52) sont constitués d'au moins deux éléments élastiques (76, 77), la tension de l'élément le plus près du matelas absorbant étant supérieure à la tension de l'élément le plus éloigné dudit matelas absorbant.

26. Culotte selon la revendication 25, dans laquelle le rapport entre la tension de l'élément le plus proche (77) et celle de l'élément le plus éloigné (76) est comprise entre environ 4 : 3 à environ 3 : 2.

27. Culotte selon la revendication 1, dans laquelle la tension desdits premiers et seconds moyens élastiques (52) est comprise entre environ 165 et environ 220 grammes.

28. Culotte selon la revendication 1, dans laquelle la tension du troisième moyen élastique (53) est comprise entre environ 330 et environ 450 grammes.

29. Culotte selon la revendication 1, dans laquelle ledit matelas absorbant (18) est sensiblement absent des panneaux avant et arrière transversalement au-delà d'une ligne longitudinale (258, 260) s'étendant depuis les bords transversaux extérieurs de ladite partie d'entre-jambes.

30. Culotte selon la revendication 1, dans laquelle ladite couverture extérieure (17) comprend un matériau étirable.

31. Culotte selon la revendication 1, dans laquelle ledit matelas absorbant (18) est constitué d'un matériau ayant une résistance à la traction à sec comprise entre environ $250 \cdot 10^{-7}$ J/cm$^2$ et environ $550 \cdot 10^{-7}$ J/cm$^2$.

FIG.1

FIG.2

1

Fig. 3

Fig. 4

3

*FIG. 5*

*FIG. 6*

FIG. 7

FIG. 8

FIG. 9

FIG. 10

FIG. 11

FIG. 12